# EUROPÄISCHE PATENTSCHRIFT

(11) **EP 1 475 626 B1**
(45) Veröffentlichungstag und Bekanntmachung des Hinweises auf die Patenterteilung: **18.07.2007**
(21) Anmeldenummer: 04000191.9
(22) Anmeldetag: 08.01.2004
(51) Int. Cl.: G01N 3/30

(54) **Verfahren und Vorrichtung zur Messung der Bildsamkeit von Materialien wie keramischen Rohstoffen und Massen**
Apparatus and method to measure the plasticity of materials like ceramic raw material and ceramic mass
Appareil et méthode pour mesurer la plasticité de matériaux comme les matières premières céramiques et céramiques

(30) Priorität: 07.05.2003 DE 10320578; 07.06.2003 DE 10325958
(43) Veröffentlichungstag der Anmeldung: 10.11.2004
(73) Patentinhaber: Brabender GmbH & Co. KG, 47055 Duisburg (DE)
(72) Erfinder: Ebert, Rolf, Prof. Dr.- Ing., D-91207 Lauf/Pegn. (DE)
(74) Vertreter: Röther, Peter

(56) Entgegenhaltungen:
- EP-A- 1 061 353
- DE-U- 20 100 489
- US-A- 6 161 422
- US-A1- 2002 053 232
- PATENT ABSTRACTS OF JAPAN Bd. 1998, Nr. 14, 31. Dezember 1998 (1998-12-31) & JP 10 260123 A (BRIDGESTONE CORP; TOYO SEIKI SEISAKUSHO:KK), 29. September 1998 (1998-09-29)

## Beschreibung

Die Erfindung betrifft ein Verfahren zur Messung der Bildsamkeit (Plastizität) von Materialien wie keramischen Rohstoffen und Massen gemäß dem Oberbegriff des Patentanspruchs 1. Weiterhin betrifft die Erfindung eine Vorrichtung zur Durchführung des Verfahrens.

Unter Bildsamkeit (Plastizität) eines keramischen Rohstoffes oder einer keramischen Masse versteht man das Vermögen, sich unter der Einwirkung äußerer Kräfte zu verformen, ohne daß dabei der Zusammenhalt der Teilchen verloren geht. Ein weiteres Merkmal der Bildsamkeit liegt darin, daß eine irreversible Verformung erst dann eintritt, wenn die äußere Kraft einen Mindestwert, den sogenannten Anlasswert (yield value) überschritten hat. Kräfte unterhalb des Anlasswertes verursachen nur eine elastische, reversible Verformung.

Bei Ton-Wasser-Gemischen findet man bildsame Verformung bei Wassergehalten von etwa 20 bis 25 %.

Der Anlasswert hat ganz wesentliche praktische Bedeutung. Er verhindert, daß sich der bereits geformte keramische Rohling unter seinem Eigengewicht deformiert.

Die Erfahrung lehrt, daß mit steigender Verformungsgeschwindigkeit die Bildsamkeit ansteigt. In der Praxis wird dieser Effekt genutzt, indem man mit möglichst hoher Geschwindigkeit formt.

### Methoden der Bildsmankeit:

Für den praktischen Betrieb sind im allgemeinen zwei Eigenschaften einer bildsamen Masse von Bedeutung:
- Die Kraft, die zur Verformung aufzuwenden ist und
- die maximal erreichbare Verformung bis zum Auftreten von Rissen.

Die Meßmethoden der Bildsamkeit lassen sich unterteilen in
. Methoden, bei denen man eine Größe misst, der man einen Bezug zur Bildsamkeit unterstellt und
. Methoden, bei denen versucht wird, unmittelbar Aufschluss über die Bildsamkeit zu erhalten.
   . Bei der **Pfefferkorn-Methode** wird auf einen Massezylinder von 40 mm Höhe (hₒ) und 33 mm 0̸ eine Platte mit definiertem Gewicht von 1192 g aus einer Höhe von 186 mm fallengelassen, wobei eine Stauchung zur Höhe h, auftritt. Als Plastizitätszahl nach Pfefferkorn gilt der Wassergehalt, bei dem das Stauchverhältnis hₒ:h₁ = 3,3:1 beträgt. Der Wassergehalt, bei dem ein Stauchverhältnis von hₒ:h₁ = 2,5:1 erreicht ist, wird als Anmachwasserbedarf bezeichnet. Während bei der Pfefferkorn-Methode die Bildsamkeit bei hoher Verformungsgeschwindigkeit gemessen wird, nutzt man bei der **Methode nach Dietzel** das gleiche Gerät, staucht den Massezylinder aber langsam bis zur Rissbildung. Als Maß gilt die Stauchung in Prozent der ursprünglichen Höhe.
   . Beim **Atterberg-Verfahren,** international von den Bo-denkundlern benutzt, werden zwei Feuchtigkeitsgehalte bestimmt, die Grenzwerte des plastischen und des flüssigen Zustandes darstellen. Der Grenzwert des plastischen Zustandes ist die Ausrollgrenze, unterhalb der sich eine Masse nicht mehr zu dünnen Strängen ausrollen läßt, ohne krümelig zu werden. Der Grenzwert des flüssigen Zustandes ist die Fließgrenze, bei der ein Einschnitt in die Masse durch Klopfen zusammenfließt. Als Maß für die Bildsamkeit dient die Breite des Wassergehalt-Bereiches zwischen den Grenzwerten.
   . Aufbauend auf dieser Methode gilt als **Plastizitätszahl nach Rieke** der Bereich zwischen der Ausrollgrenze und dem Anmachwasserbedarf, der hier so definiert ist, daß die Masse gerade nicht mehr an den Händen klebt.
   . Nach **Cohn** bestimmt man den Wassergehalt, bei dem ein genormter, belasteter Stab in einer vorgegebenen Zeit in eine bestimmte Tiefe in die Masse eindringt.
   . **Norton** bzw. **Baudran** verwenden die Torsionsprüfung für die Bestimmung einer Maßzahl für die Bildsamkeit. Das Produkt aus Anlasswert und maximaler Verformung wird als "workability" bezeichnet. Es erreicht bei einem bestimmten Wassergehalt ein Maximum.
   . Umgekehrt verwendet **Haase** als Maß für die Bildsamkeit den Quotienten aus Zerreißfestigkeit und Deformationsdruck. Seine Überlegungen gehen davon aus, daß eine Masse umso bildsamer ist, je größer der Zusammenhalt der Teilchen (= Zerreißfestigkeit) ist, je kleiner aber die Kräfte zum gegenseitigen Verschieben der Teilchen sind, d.h. je niedriger die Viskosität der Masse ist.
   . Auf einer ähnlichen Überlegung beruht die Methode nach **Hofmann** und **Linseis**. Hier wird als Maß für die Bildsamkeit der Quotient aus Zerreißfestigkeit und Anlasswert verwendet. Der Anlasswert wird charakterisiert durch den Auspressdruck, mit dem die Masse durch eine Düse gefördert werden kann.
   . Bei der Ermittlung der Bildsamkeit mit dem **Brabender-Plastographen** wird das Pulver bei kontinuierlich steigendem Wassergehalt in einer Knetkammer durchmischt. Registriert wird das Drehmoment des Antriebsmotors der Knetarme, d.h. der Widerstand des Pulver-Flüssigkeits-Systems gegen die Verformung. Als Maß für die Bildsamkeit gelten die Höhe des Drehmoments im Kurvenmaximum sowie die Steilheit der Flanken des Maximums. Aus der Kurve kann darüber hinaus der Wassergehalt beim maximalen Drehmoment entnommen werden.
   . Für eine theoretische Behandlung der Bildsamkeit mit am aussagefähigsten ist ein von **Ashbury** angegebener zyklischer Belastungsversuch (Torsion), bei dem die einwirkende Spannung zwischen den Maximalwerten +t und -t mit einer Periodendauer von ca. 1 min schwankt. Registriert wird die sich einstellende Verformung e. Der Flächeninhalt der Hysteresekurven ist ein Maß für die aufzuwendende Verformungsarbeit.

Die Aussagefähigkeit und Akzeptanz der Methoden ist zum Teil durch einen hohen messtechnischen Aufwand, zum Teil durch die beschränkte Genauigkeit und nicht vorhandene Personen-Neutralität beschränkt. Die elastischen Eigenschaften der Probe werden mit Ausnahme des Brabender-Plastographen (Messkneter) und der Torsions-Methode nicht erfasst.

Darüber hinaus ist aus der Entgegenhaltung EP 1 061 353 A2 ein Verfahren bekannt, bei dem eine Vielzahl von Varianten einer Zugprüfung, insbesondere runder oder metallischer Prüfkörper beschrieben werden. Dabei handelt es sich um Scherung, Biegung und Torsion. Die Möglichkeit eines Stauchtest wird ebenfalls erwähnt.

Die Konstruktion ist so ausgelegt, dass alle Übergänge von einer Impulsbelastung im langsamen Lastanstieg bis zur statischen Belastung verfahren werden können. Die bevorzugte Ausrichtung auf eine Zugprüfung ist erkenntlich durch die Konstruktion des Kraftaufnehmers mit zwei Einzelsensoren und einer Senke dazwischen, um die Zugproben aufzunehmen. Es handelt sich hier um ein Gerät zur Prüfung eines fertigen Werkstoffs bezüglich der Einsetzbarkeit in Konstruktionen und nicht wie bei der Erfindung um die Prüfung eines Zwischenzustandes, der die Prüfung der Eignung eines Materials zur weiteren Verarbeitung zum Ziel hat. Der endgültige Werkstoff (beispielsweise Keramik) existiert zu diesem Zeitpunkt noch nicht.

Darüber hinaus geht aus der JP 10 260 123 A ebenfalls die Messung der Eigenschaften eines elastischen Prüfkörpers hervor. Es handelt sich um ein Pendelschlagwerk ähnlich der Kerbschlagprüfung bei Metallen, wobei die Stärke des Rückschwingens des Pendels als Maß für die Eigenschaften des Werkstoffs registriert wird. Wesentlich ist, dass bei dieser Art der Prüfmethoden der Last waagerecht im unteren Totpunkt des Pendels erfolgt.

Erfindungsgemäß erfolgt der Aufprall der Last aber immer vertikal.

Der Erfindung liegt daher die Aufgabe zugrunde, ein Verfahren der eingangs genannten Art so zu verbessern, daß es unabhängiger ist von individuellen Beurteilungen und die Genauigkeit der Messungen verbessert wird.

Die Erfindung löst diese Aufgabe gemäß dem kennzeichnenden Teil des Patentanspruchs 1 dadurch, dass der Probenkörper auf eine Kraftmessvorrichtung aufgesetzt wird, wobei der Kraftaufnehmer in Schwingungen versetzbar ist und beim Verformungsvorgang zusätzlich der zeitliche Verlauf der vom Probenkörper beim Verformungsvorgang aufgebrachten Reaktionskraft gemessen wird, wobei die Schwingungen des Kraftaufnehmers dem Kraftsignal überlagert werden, das Dämpfungsverhalten der Schwingungen durch den Probenkörper selbst erfasst wird, der Messvorgang bis zum Verformungsende der Probe durchgeführt wird und die Messwerte (Weg und Kraft) einem Rechner zur Verarbeitung und Auswertung zugeführt werden.

Somit wird auf der Basis der Pfefferforn-Methode eine neue, wesentlich erweiterte Methode vorgestellt, die zum einem die elastischen Eigenschaften berücksichtigt und zum anderen durch die Darstellung der Ergebnisse nicht nur den Endpunkt eines Meßvorgangs als Ergebnis zur Verfügung stellt, sondern den gesamten Ablauf der Verformung beginnend mit hoher Verformungsgeschwindigkeit sichtbar macht und dadurch eine sehr viel weitergehende Charakterisierung der plastischen Werkstoffeigenschaften ermöglicht.

Die Erfindung löst die obengenannte Aufgabe auch durch eine Vorrichtung, auf der das eben angegebene Verfahren ausgeführt wird.

Die Vorrichtung besteht aus einem geführten Fallgewicht (beispielsweise wie bei der Pfefferkorn-Methode mit einem Gewicht von 1192 g), das aus einer einstellbaren Höhe (zwischen 100 und 200 mm) auf die (im Normall zylindrische) Probe fällt und diese staucht, beispielsweise auf ca. 1/3 seiner ursprünglichen Höhe. Die Probe steht auf einer Kraftmesseinheit (beispielsweise eine Kraftmessdose). Diese Kraftmessdose kann in sich selbst federnd sein oder alternativ selbst nicht federnd aber auf einem separaten Federsystem mit Wegaufnehmer gelagert sein.

Aus dem Diagramm 1 ergibt sich ein typischer Verlauf der Messwerte, zum einen die der Wegmessung und zum anderen die der Kraftmessung.

### Hierbei bedeuten:

t_{D} Deformationszeit (ms)
F_{P} Peak/Kraftsignal (N)
s Deformation (mm)
Wₑ Elastische Verformungsenergie (Nmm)
W_{P} Plastische Verformungsenergie (Nmm)
m₁ Kraftanstieg (elastisch) (N/ms)
m₂ Kraftanstieg (plastisch) (N/ms)
m₃ Kraftsignalabfall (N/ms)

Wie sich aus diesem Diagramm ergibt, ist die Anfangsverformung von hoher Geschwindigkeit und geht dann in eine Verformung mit geringerer Geschwindigkeit über und endet schließlich bei der Endverformung (horizontaler Bereich).

Die Kraftmessung ergibt, daß hier anfangs abhängig vom Anlaßwert der Probe ein mehr oder weniger hoher Kraftanstieg zu beobachten ist, wodurch die elastische Verformung bewirkt wird. Der Kraftsignalverlauf geht nach einem in etwa linearen Anstieg in einen je nach Dämpfungsverhalten der Probe mehr oder weniger wellenförmigen Anstieg mit geringerer Steigung über. Dieser Bereich repräsentiert die plastische Verformung.

Die Steigung m₁ gibt den elastischen Kraftanstieg und die Steigung m₂ den plastischen Kraftanstieg an.

Die Fläche unterhalb des linearen Anstiegs wₑ gibt die elastische Verformungsenergie und die Fläche unterhalb der wellenförmigen Kurve wₚ gibt die plastische Verformungsenergie an.

Gemessen wird über einen Zeitraum t_{D}, der als Deformationszeit in Millisekunden bezeichnet wird.

Das absolute Maximum der Kraftsignalkurve F_{P} gibt die maximal wirkende Kraft an, wonach dann im linear abfallenden Bereich m₃ die Kraft auf Null zurückgeht (m₃).

Somit ergibt sich der Signalverlauf an der Kraftmessdose aus dem durch die plastischen und elastischen Eigenschaften des Probenmaterials modellierten Impuls des Fallgewichts. Aus dem Kurvenverlauf lassen sich charakteristische Merkmale des plastischen (bildsamen) Verhaltens der Probe direkt herauslesen. Das Signal enthält Informationen über die plastischen Eigenschaften der Proben in Form des Dämpfungsverhaltens und des Impulsdurchgangs durch das Probenmaterial. Zusätzlich wird der Kraftmessaufnehmer durch den Aufprall zu Schwingungen angeregt, die dem Kraftsignal überlagert sind. Die Ausprägung dieser Schwingungen ist durch folgende Einflussgrößen bestimmt:
- Messbereich und Federeigenschaften des Messaufnehmers,
- Härte der Probe
- Dämpfungsverhalten bzw. elastische Eigenschaften des Probenmaterials.

Eine Verarbeitungssoftware interpretiert den Kurvenverlauf und stellt Zahlenwerte zur Verfügung, die die Plastizität des Probenmaterials kennzeichnen. Über den Signalverlauf kann ein Toleranzbereich gelegt werden, der zu einer automatisierten "pass-fail" - Entscheidung genutzt werden kann.

Die Führung des Fallgewichts kann eine Linearführung sein, in der das Fallgewicht im freien Fall auf die Probe fällt oder aber zwangsgesteuert mit einer konstanten, beschleunigten, verzögerten oder oszillierenden Geschwindigkeit.

Das Fallgewicht kann allerdings auch an einem langen Hebel um eine Drehachse herum bewegt werden, wobei die Drehachse höhenverstellbar sein kann.

Bei der Führung kann es sich allerdings auch um einen langen Hebel mit Parallelogrammführung oder um ein Scherensystem handeln.

Zur Signalaufnahme wird ein relative oder auch absolute Messwerte messender Wegsensor oder ein ebensolcher Winkelsensor (beim Hebelsystem) eingesetzt.

Alle denkbaren Weg- und Winkelsensoren (Potentiomer, auf dem Hall-Effekt beruhend, optisch arbeitend, inkremental arbeitend) können eingesetzt werden.

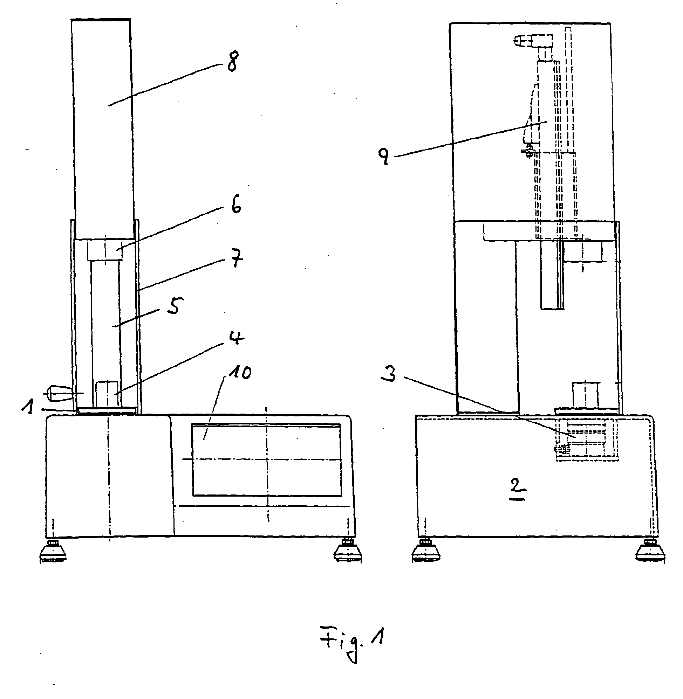

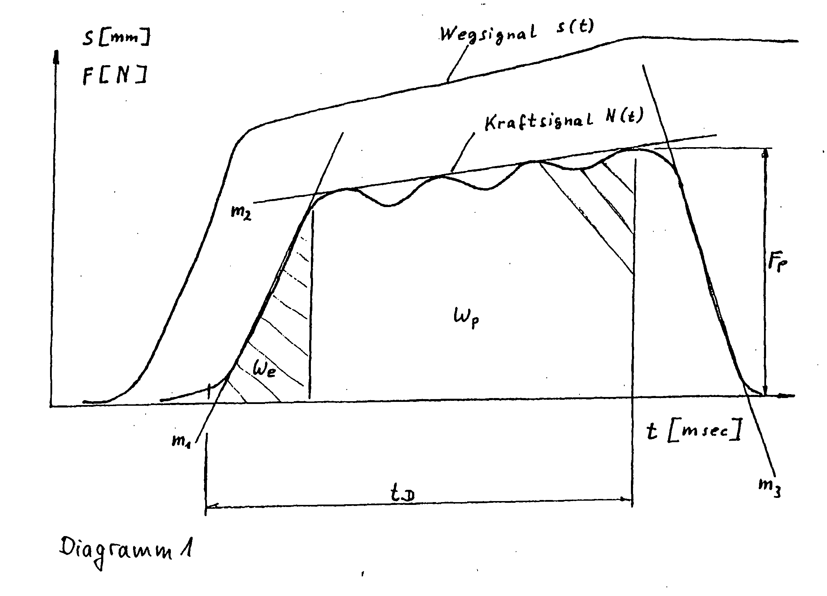

In der Figur 1 ist die Vorrichtung prinzipiell dargestellt, und zwar sowohl in Seiten- als auch in Vorderansicht.

Auf einer Platte 1, die auf einer in einem Gehäuse 2 gelagerten Kraftmessdose 3 aufsitzt, ist ein Probenkörper 4 aus einer keramischen Masse angeordnet. Lotrecht über diesem Probenkörper 4 befindet sich eine Führung 5 für ein Fallgewicht 6. Führung und Fallgewicht befinden sich einem Gehäuse, das aus einer Schutzhaube 7 und einer oberen Haube 8 besteht. In der oberen Haube 8 ist ein Wegsensor 9 zur Detektierung der Bewegung des Fallgewichts 6 angeordnet.

Im unteren Gehäuse 2 befindet sich ein Rechner, dem die Messerte des Wegsensors 9 bzw. der Kraftmessdose 3 zugeleitet werden und dort verarbeitet werden. Angezeigt werden die Ergebnisse auf einem Bildschirm 10 (siehe Diagramm 1).

Im vorliegenden Ausführungsbeispiel beträgt das Gewicht des Fallgewichts 1192 g. Die Fallhöhe ist einstellbar zwischen 100 und 200 mm.

## Patentansprüche

1. Verfahren zur Messung der Bildsamkeit (Plastizität) von Materialien wie keramischen Rohstoffen und Massen, bei dem auf einen Probenkörper ein Gewicht einwirkt und ein die Verformung des Probenkörpers wiedergebendes Wegsignal gemessen wird,
**dadurch gekennzeichnet,**
**dass** der Probenkörper auf eine Kraftmessvorrichtung (3) aufgesetzt wird, wobei der Kraftaufnehmer in Schwingungen versetzbar ist und beim Verformungsvorgang zusätzlich der zeitliche Verlauf der vom Probenkörper beim Verformungsvorgang aufgebrachten Reaktionskraft gemessen wird, wobei die Schwingungen des Kraftaufnehmers dem Kraftsignal überlagert werden, das Dämpfungsverhalten der Schwingungen durch den Probenkörper selbst erfasst wird, der Messvorgang bis zum Verformungsende der Probe durchgeführt wird und die Messwerte (Weg und Kraft) einem Rechner zur Verarbeitung und Auswertung zugeführt werden.

2. Verfahren nach Anspruch 1,
**dadurch gekennzeichnet,**
**dass** das Gewicht aus einer bestimmten Höhe auf den Probenkörper fallengelassen wird.

3. Verfahren nach Anspruch 1 oder 2,
**dadurch gekennzeichnet,**
**dass** das Gewicht im freien Fall auf den Probenkörper auftrifft.

4. Verfahren nach Anspruch 1 oder 2,
**dadurch gekennzeichnet,**
**dass** das Gewicht mit einer geregelten Geschwindigkeit auf den Probenkörper auftrifft.

5. Vorrichtung zur Durchführung des Verfahrens nach Anspruch 1 bestehend aus einem Probentisch (1), einer über dem Probentisch angeordneten Führung für ein auf einen Probenkörper einwirkendes Gewicht (6) sowie einem Wegsensor (9), der zur Messung des Weges des Gewichts (6) vorgesehen ist, wobei der Probentisch (1) auf einer Kraftmessvorrichtung angeordnet ist,
**dadurch gekennzeichnet,**
**dass** die Kraftmessvorrichtung eine in Schwingung versetzbare Kraftmessdose (3) ist, die selbstfedernd und/oder auf einem separaten Federsystem mit Wegaufnehmer gelagert ist und die Kraftmessvorrichtung die Reaktionskraft messen kann und mit einem Rechner verbunden ist, der das Dämpfungsverhalten der Schwingungen durch den Probenkörper gleichzeitig mit dem Weg und der Kraft von Anfang bis Ende des Verformungsvorgangs erfassen kann.

6. Vorrichtung nach Anspruch 5,
**dadurch gekennzeichnet,**
**dass** der Wegsensor (9) und die Kraftmessvorrichtung (3) über Signalleitungen mit einem Rechner verbunden sind.

7. Vorrichtung nach einem der Ansprüche 5 oder 6,
**dadurch gekennzeichnet,**
**dass** das Gewicht in einer Linearführung geführt ist.

8. Vorrichtung nach einem der Ansprüche 5 oder 6,
**dadurch gekennzeichnet,**
**daß** das Gewicht an einem um eine Drehachse verdrehbaren langen Hebel angeordnet ist.

9. Vorrichtung nach Anspruch 8,
**dadurch gekennzeichnet,**
**dass** die Drehachse höhenverstellbar ist.

10. Vorrichtung nach einem der Ansprüche 5 oder 6,
**dadurch gekennzeichnet,**
**dass** das Gewicht an einem Hebel mit Parallelogrammführung angeordnet ist.

11. Vorrichtung nach einem der Ansprüche 5 oder 6,
**dadurch gekennzeichnet,**
**dass** das Gewicht an einem Scherensystem angeordnet ist.

## Claims

1. A process for the measurement of the plasticity of materials such as ceramic raw materials and substances in which a weight acts on a sample body and a path signal which records the distortion of the sample body is measured,
**characterised in that**
the sample body is placed on a strength measuring device (3) in which the strength recorder can be converted into oscillations and in addition during the distortion procedure the temporal path of the strength of reaction incurred during the distortion procedure is measured while the oscillations of the strength recorder are superimposed on the power signal, the damping behaviour of the oscillations is itself collated by the sample body, the measurement procedure is carried out until the final distortion of the sample and the measurement values (path and strength) are entered into a computer for processing and evaluation.

2. A process in accordance with Claim 1,
**characterised in that**
the weight is dropped on to the sample body from a specific height.

3. A process in accordance with Claim 1 or 2,
**characterised in that**
the weight strikes the sample body in free fall.

4. A process in accordance with claim 1 or 2,
**characterised in that**
the weight strikes the sample body at a regulated velocity.

5. A device to carry out the process in accordance with claim 1, comprising a test bench (1), a keyway fitted above the test bench for a weight (6) which acts on the sample body and a path sensor (9) which is provided for the measurement of the path of the weight (6), during which the test bench (1) is fitted to a strength measurement device,
**characterised in that**
the strength measurement device is a strength measurement box (3) which can be converted into oscillations and which is self-sprung and/or fitted on to a separate spring system with a path recorder and the strength measurement device can measure the strength of reaction and is connected to a computer which can collate the damping behaviour of the oscillations by the test body at the same time as the path and strength from the beginning to the end of the distortion process.

6. A device in accordance with Claim 5,
**characterised in that**
the path sensor (9) and the strength measuring device (3) are connected to a computer by signal circuits.

7. A device in accordance with Claim 5 or 6,
**characterised in that**
the weight is led in a linear keyway.

8. A device in accordance with Claim 5 or 6,
**characterised in that**
the weight is arranged on a long lever which can be twisted round a fulcrum.

9. A device in accordance with Claim 8,
**characterised in that**
the fulcrum is adjustable in height.

10. A device in accordance with one of the Claims 5 or 6,
**characterised in that**
the weight is arranged on a lever with a parallelogram keyway..

11. A device in accordance with one of the Claims 5 or 6,
**characterised in that**
the weight is arranged on a scissors system.

## Revendications

1. Procédé pour mesurer la plasticité de matériaux tels que matières premières et pâtes céramiques, dans lequel un poids agit sur une éprouvette et un signal de course traduisant la déformation de l'éprouvette est mesuré, **caractérisé en ce qu'**on place l'éprouvette sur un dispositif de mesure de force (3), tandis qu'on fait osciller le capteur de force et que lors du processus de déformation on mesure en outre la variation dans le temps de la force de réaction appliquée par l'éprouvette lors du processus de déformation, les oscillations du capteur de force étant superposées au signal de force, le comportement à l'amortissement des oscillations par l'éprouvette elle-même étant relevé, l'opération de mesure étant exécutée jusqu'à la fin de la déformation de l'éprouvette et les valeurs de mesure (course et force) étant envoyées à un ordinateur pour traitement et interprétation.

2. Procédé selon la revendication 1, **caractérisé en ce qu'**on laisse tomber le poids depuis une hauteur déterminée sur l'éprouvette.

3. Procédé selon la revendication 1 ou 2, **caractérisé en ce que** le poids parvient en chute libre sur l'éprouvette.

4. Procédé selon la revendication 1 ou 2, **caractérisé en ce que** le poids parvient avec une vitesse régulée sur l'éprouvette.

5. Dispositif pour la mise en oeuvre du procédé selon la revendication 1, constitué d'une table à éprouvettes (1), d'un guide disposé au-dessus de la table à éprouvettes et d'un poids (6) agissant sur une éprouvette ainsi que d'un capteur de course (9) qui est prévu pour la mesure de la course du poids (6), la table à éprouvettes (1) étant disposée sur un dispositif de mesure de force, **caractérisé en ce que** le dispositif de mesure de force est une boîte dynamométrique (3) que l'on peut faire osciller, qui est auto-oscillante et/ou montée sur un système élastique séparé avec capteur de course, et le dispositif de mesure de force peut mesurer la force de réaction et est relié à un ordinateur qui peut en même temps relever le comportement à l'amortissement des oscillations par l'éprouvette ainsi que la course et la force depuis le début jusqu'à la fin du processus de déformation.

6. Dispositif selon la revendication 5, **caractérisé en ce que** le capteur de course (9) et le dispositif de mesure de force (3) sont reliés à un ordinateur par des lignes de signaux.

7. Dispositif selon l'une des revendications 5 ou 6, **caractérisé en ce que** le poids est guidé dans un guide linéaire.

8. Dispositif selon l'une des revendications 5 ou 6, **caractérisé en ce que** le poids est disposé sur un long levier qui peut tourner autour d'un axe de rotation.

9. Dispositif selon la revendication 8, **caractérisé en ce que** l'axe de rotation est réglable en hauteur.

10. Dispositif selon l'une des revendications 5 ou 6, **caractérisé en ce que** le poids est disposé sur un levier avec guide à parallélogramme.

11. Dispositif selon l'une des revendications 5 ou 6, **caractérisé en ce que** le poids est disposé sur un système à ciseaux.
